# FASCICULE DE BREVET EUROPEEN

(11) **EP 0 655 221 B1**
(45) Date de publication et mention de la délivrance du brevet: **09.02.2000**
(21) Numéro de dépôt: 94402586.5
(22) Date de dépôt: 16.11.1994
(51) Int. Cl.: A61B 5/103, G01N 21/47, G01J 3/50

(54) **Tête de mesure colorimétrique, et procédé pour déterminer la couleur interne d'un matériau non opaque**
Kolorimetrischer Messkopf und Verfahren zur Farbbestimmung des Inneren eines lichtdurchlässigen Materials
Colorimetrical measuring head, and method for determining the internal colour of a non opaque material

(30) Priorité: 26.11.1993 FR 9314165
(43) Date de publication de la demande: 31.05.1995
(73) Titulaire: L'OREAL, 75008 Paris (FR)
(72) Inventeur: Caisey, Laurence, F-94400 Vitry-Sur-Seine (FR); Bauer, Daniel, F-93340 Le Raincy (FR)
(74) Mandataire: Peuscet, Jacques

(56) Documents cités:
- EP-A- 0 530 818
- EP-A- 0 604 276
- WO-A-88/05284
- WO-A-89/08428
- DE-A- 4 031 320
- FR-A- 2 658 410
- GB-A- 2 022 244
- US-A- 3 947 088
- US-A- 4 213 462
- US-A- 4 816 670

## Description

L'invention est relative à une tête de mesure colorimétrique, pour déterminer la couleur interne d'un matériau non opaque, en particulier la couleur interne de la peau humaine.

Jusqu'à présent, l'aspect des matériaux a été appréhendé avec des appareils de mesure optiques permettant essentiellement l'étude des propriétés réfléchissantes de la surface de ces matériaux.

FR-A-2 650 890 concerne un appareil optique destiné à permettre l'évaluation de la brillance d'une surface. Cet appareil relativement complexe fait intervenir des filtres polariseur et analyseur. En outre, la mesure est effectuée en supprimant la composante de couleur interne.

Dans tous ces dispositifs optiques, on privilégie les flux lumineux issus de la surface du matériau à étudier. Ces dispositifs optiques sont donc très utiles pour l'étude de surfaces opaques.

Mais il existe dans la nature, ou l'industrie, des objets constitués de matériaux complexes d'un point de vue optique car à la fois plus ou moins transparents, sélectivement absorbants, diffusants, hétérogènes.

Pour de tels matériaux non opaques, les indications fournies par les appareils de mesure optiques classiques ne permettent pas de bien classer les matériaux. En particulier, ces dispositifs classiques de mesure peuvent donner, pour deux matériaux non opaques d'aspect visuel très différent, des indications similaires ne permettant pas de faire une distinction entre les deux matériaux.

On connaît par le document DE-A-4 031 320, un dispositif d'examen optique conforme au préambule de la revendication 1.

L'invention a pour but, surtout, de fournir une tête de mesure colorimétrique permettant de déterminer la couleur interne d'un matériau non opaque et notamment d'effectuer une distinction entre deux matériaux non opaques, d'aspect visuel différent, et pour lesquels, avec un appareil colorimétrique classique, aucune différence ne serait enregistrée.

L'invention a également pour but de fournir une tête de mesure colorimétrique de construction simple et économique propre à prendre en compte essentiellement la composante de couleur interne et sans que la composante de brillance de la surface n'entre en jeu.

Selon l'invention, une tête de mesure colorimétrique, pour déterminer la couleur interne d'un matériau non opaque, en particulier la couleur interne de la peau humaine, comporte un système optique émetteur de lumière, en direction du matériau à étudier, séparé par une paroi opaque d'un système optique récepteur de la lumière qui a pénétré dans le matériau et qui est renvoyée par celui-ci, et est caractérisée par le fait que la surface externe de la tête tournée vers le matériau à étudier est continue et adaptée à la surface du matériau de manière à épouser cette surface soit par application directe contre ladite surface du matériau, soit avec un mouillage optique, et qu'elle comporte une paroi opaque extérieure coulissante entre une position en saillie pour permettre une mesure classique de couleur de surface, et une position effacée où l'extrémité de la paroi opaque est située sur la surface continue de l'extrémité de la tête, de manière à s'appliquer contre le matériau et à permettre la mesure de la couleur interne de ce matériau non opaque.

Avec cette tête de mesure colorimétrique, la lumière reçue par le récepteur provient de l'intérieur du matériau, tandis que la lumière éventuellement renvoyée par la surface du matériau ou venant directement de l'émetteur ne peut atteindre le récepteur en raison de la paroi opaque de séparation.

Si cette tête optique est posée sur une surface opaque, même très blanche, aucune lumière n'est transmise de l'émetteur au détecteur. Il en est de même si la tête de mesure est posée sur un matériau parfaitement transparent.

Cette tête de mesure permet de déterminer la quantité de lumière qui passe dans le matériau, en particulier dans la peau, et qui revient dans le détecteur, grâce à des "conducteurs optiques" formés par le matériau à étudier. On peut ainsi apprécier la "couleur interne" du matériau et distinguer en particulier une "peau épaisse, opaque" d'une "peau translucide".

De préférence, la tête optique est entourée d'une paroi opaque protégeant la tête contre la lumière extérieure parasite.

La tête optique peut avoir une forme cylindrique, de préférence de révolution, l'émetteur et le récepteur étant coaxiaux et séparés par une paroi cylindrique opaque coaxiale. Une paroi opaque extérieure entoure l'ensemble.

L'émetteur peut être agencé pour émettre un faisceau de lumière parallèle ou pour émettre de la lumière diffuse, à l'aide d'un verre diffusant placé devant une source lumineuse, notamment une lampe flash annulaire.

De même, le détecteur peut assurer soit une détection multi-directionnelle soit une détection collimatée.

L'émetteur et/ou le détecteur peut comprendre une fibre optique dont une extrémité est située sur la surface externe de la tête destinée à être appliquée contre le matériau.

La détection collimatée peut être réalisée à l'aide d'un système collimaté avec lentille convergente et diaphragme au foyer de la lentille, ou d'un système de détection sélective avec une fibre optique.

La détection multi-directionnelle peut être réalisée avec une sphère d'intégration comportant une ouverture destinée à être placée sur la zone du matériau à étudier, et une cellule de détection prévue sur la surface de la sphère d'intégration.

Le système de détection peut petre composé de cellules trichromatiques, d'un spectrophotomètre ou simplement d'un photomètre.

L'invention est également relative à un procédé pour déterminer la couleur interne d'un matériau non opaque, caractérisé par le fait qu'on applique contre ce matériau une tête de mesure colorimétrique présentant une surface qui épouse la surface du matériau, la tête de mesure comportant un émetteur de lumière séparé d'un récepteur par une paroi opaque.

L'invention consiste, mises à part les dispositions exposées ci-dessus, en un certain nombre d'autres dispositions dont il sera plus explicitement question ci-après à propos d'exemples de réalisation décrits avec référence aux dessins ci-annexés, mais qui ne sont nullement limitatifs.
La figure 1, de ces dessins, est une vue schématique en coupe verticale axiale d'une tête de mesure colorimétrique, selon l'invention, appliquée contre un matériau non opaque à étudier.
La figure 2 est une coupe suivant II-II de la figure 1.
La figure 3 est un schéma simplifié d'un émetteur de lumière produisant une émission dirigée.
La figure 4 est une coupe schématique d'un émetteur de lumière produisant une émission diffuse.
La figure 5 est une coupe verticale axiale d'une variante de réalisation de la tête de mesure colorimétrique.
La figure 6 est une coupe schématique d'une autre variante avec un récepteur constitué par une fibre optique.
La figure 7 est un schéma d'une autre variante avec détection collimatée à l'aide d'une lentille convergente.
La figure 8 est une coupe verticale axiale d'une variante de tête de mesure colorimétrique avec sphère d'intégration au niveau du détecteur.
La figure 9 est une coupe schématique d'une variante de réalisation de tête optique avec paroi opaque extérieure coulissante, dans sa position effacée.
La figure 10, enfin, montre semblablement à la figure 9 la tête optique alors que la paroi opaque extérieure est en saillie.

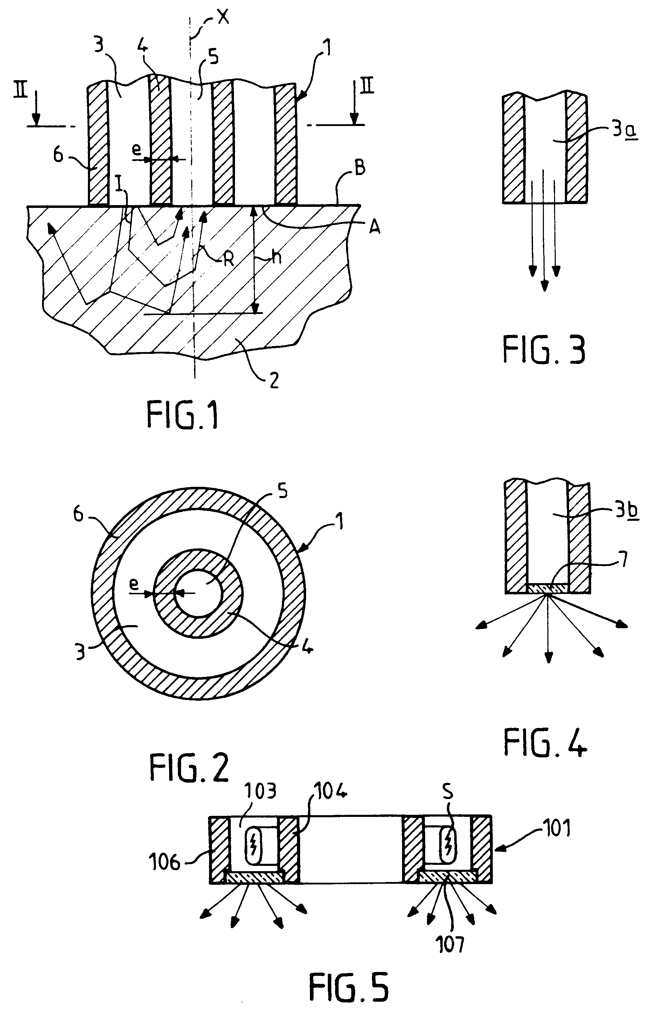

En se reportant à la figure 1, on peut voir une tête de mesure colorimétrique 1, partiellement représentée, pour déterminer la couleur interne d'un matériau 2 non opaque, en particulier constitué par la peau humaine. La tête 1 comporte un système optique 3 émetteur de lumière en direction du matériau 2 à étudier, séparé par une paroi opaque 4 d'un système optique récepteur 5.

Dans l'exemple considéré, la tête 1 a une forme cylindrique de révolution d'axe X vertical. Le système émetteur 3 a une forme annulaire tandis que le récepteur 5 est disposé suivant l'axe de la tête 1. Une paroi opaque extérieure 6, également cylindrique, entoure le récepteur annulaire 3 et l'ensemble de la tête.

L'émetteur 3 comporte une source lumineuse annulaire non visible sur la figure 1, mais semblable à la source S représentée sur la figure 5 et décrite plus loin. L'espace annulaire compris entre la source lumineuse et l'extrémité de la tête 1 appliquée contre le matériau 2 est conducteur de la lumière et peut émettre un faisceau de lumière parallèle ou un faisceau de lumière diffuse.

En particulier, l'espace annulaire de l'émetteur 3 peut comporter une couronne de fibres optiques. La figure 3 illustre schématiquement un émetteur 3a de lumière parallèle à fibre optique.

Il est également possible d'utiliser un émetteur 3b (figure 4) émettant de la lumière diffuse, par exemple à l'aide d'un verre diffusant 7 placé à l'extrémité de l'émetteur 3b tournée vers le matériau à étudier.

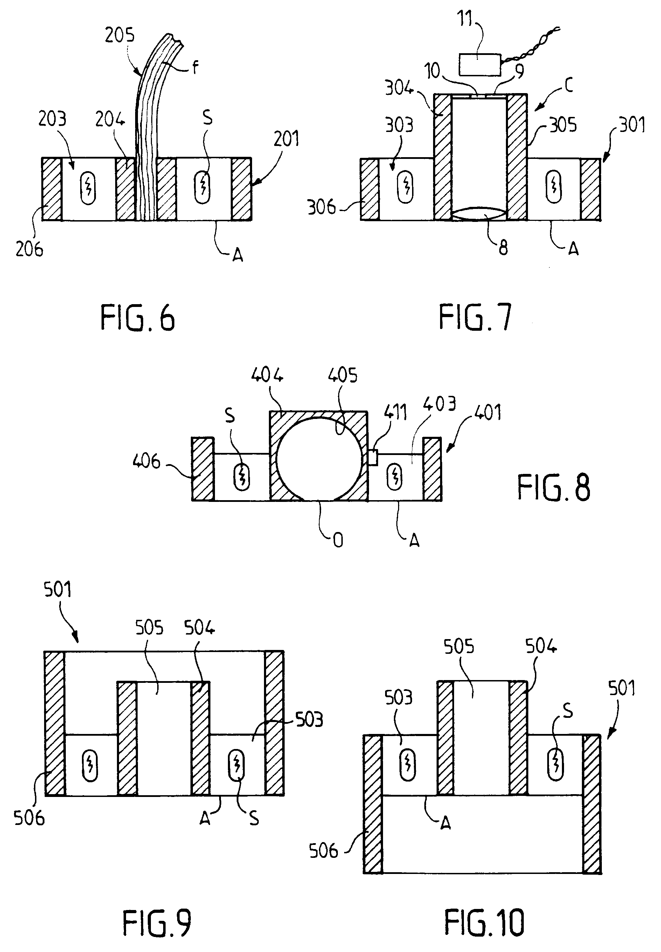

Le détecteur 5 peut assurer une détection collimatée, en lumière parallèle, comme cela sera expliqué à propos des figures 6 et 7, ou une détection multi-directionnelle. Le détecteur 5 comprend un élément photodétecteur, non représenté sur la figure 1, semblable à l'élément 11 de la figure 7, décrite plus loin.

La surface externe A de la tête 1, tournée vers le matériau 2 à étudier, est continue et adaptée à la surface du matériau de manière à épouser cette surface soit par application directe contre ladite surface B, soit avec interposition d'une goutte d'huile ou un moyen équivalent.

Dans l'exemple considéré, la surface d'extrémité A de la tête 1 est plane, orthogonale à l'axe X. Il est clair que cette surface d'extrémité A pourrait être courbe, convexe ou concave, selon la surface du matériau à étudier. Les extrémités de l'émetteur 3, de la paroi opaque 4, du récepteur 5 et de la paroi extérieure 6 sont toutes dans un même plan de sorte que lorsque la tête 1 est appliquée contre la surface B, comme illustré sur la figure 1, la lumière incidente I provenant de l'émetteur 3 ne peut passer directement vers le récepteur 5 car elle en est empêchée par la paroi 4. Le détecteur 5 ne peut recueillir que la lumière R qui, après avoir pénétré dans le matériau, est diffusée et transmise par ce matériau, définissant des sortes de "conducteurs optiques".

La profondeur h jusqu'à laquelle peut descendre dans le matériau 2 la lumière émise, qui est ensuite recueillie par le détecteur 5, dépend de plusieurs facteurs, dont l'épaisseur e de la paroi 4 séparant émetteur et détecteur. Une augmentation de l'épaisseur e provoque, dans certaines limites, une augmentation de la profondeur h des couches du matériau qui sont prises en compte pour l'appréciation de la couleur.

Dans la pratique, suivant la nature du matériau et la profondeur à laquelle on veut travailler, l'épaisseur de la paroi opaque 4 de séparation est comprise entre 0,3 mm et 10 mm (0,3 ≤ e ≤ 10 mm).

Il faut naturellement prévoir un éclairage suffisant, au niveau du système émetteur, pour prendre en compte les couches relativement profondes du matériau 2.

La paroi extérieure 6, qui forme un anneau opaque comme visible sur la figure 2, limite la surface irradiée du matériau tout en assurant une protection vis-à-vis de la lumière extérieure parasite.

Il est clair que l'on peut inverser la position de l'émetteur et du détecteur par rapport à la description donnée jusqu'à présent, c'est-à-dire que l'émetteur peut être situé suivant l'axe X de la tête tandis que le détecteur peut être annulaire.

La figure 5 illustre une variante de réalisation 101 de la tête optique avec émission diffuse. Une lampe flash annulaire S concentrique à la tête est disposée dans l'espace compris entre les parois opaques 104, 106. Un verre diffusant 107, en forme de couronne circulaire, est prévu dans la face de la tête 101 destinée à être appliquée contre le matériau à étudier.

La figure 6 illustre schématiquement une tête 201 dans laquelle le détecteur 205 comprend une fibre optique f dont l'extrémité est située dans le plan de la surface A de la tête 201 de manière à être appliquée directement contre le matériau à étudier. L'ouverture peut être variable selon la fibre optique f. La fibre optique f permet de réaliser une détection collimatée, qui donne plus d'importance à la lumière issue des profondeurs du matériau 2.

Cette détection collimatée peut être réalisée, comme illustré sur la figure 7, à l'aide d'un système collimaté C à la place de fibres optiques. Le système C comprend, au niveau du détecteur 305, une lentille convergente 8 dont la face avant est dans le plan A de la tête 301 destiné à s'appliquer contre le matériau. L'enveloppe 304 forme un cylindre dont la longueur est égale à la distance focale de la lentille 8. A l'extrémité du cylindre 304 éloignée de la lentille 8, un écran 9 est disposé avec un orifice 10 en son centre, situé au foyer de la lentille 8. Une cellule de détection 11, comprenant un élément photodétecteur, est placée derrière l'orifice 10, et reçoit un faisceau de lumière parallèle. L'orifice 10 a un diamètre en rapport avec la qualité de la collimation.

Il est à noter que, sans la lentille 8, le cylindre 304 constituerait une chambre d'intégration qui permet de recueillir la lumière sans favoriser une direction privilégiée.

La figure 8 illustre une variante de réalisation 401 de la tête optique avec détection multi-directionnelle grâce à une sphère d'intégration 405 prévue au niveau du détecteur.

La paroi de séparation 404 est constituée par une sorte de bloc opaque à l'intérieur duquel est ménagée une cavité sphérique dont la surface interne, constituant la sphère d'intégration 405, est propre à renvoyer la lumière. Cette surface, par exemple, est blanche. Une ouverture circulaire O est prévue en partie basse du bloc 404 pour permettre l'entrée de la lumière, renvoyée par le matériau à étudier, dans la sphère 405. Le plan de cette ouverture O est confondu avec le plan A de l'extrémité de la tête 401.

Un élément détecteur 411 constitué soit directement par une cellule photodétectrice, soit par l'extrémité d'une fibre optique conduisant à une cellule photodétectrice, ou par tout moyen équivalent, est prévu dans le bloc 404 pour déboucher à la surface de la sphère 405 et recueillir la lumière intégrée par la sphère 405.

La figure 9 illustre une variante de tête de mesure optique 501, selon l'invention, à "géométrie variable". On retrouve un système détecteur 505 central entouré par une paroi opaque 504. L'émetteur 503 est entouré par une paroi opaque 506 coulissante dont la longueur axiale est supérieure à celle du détecteur 503.

Dans une position effacée représentée sur la figure 9, la paroi extérieure 506 a son extrémité inférieure située dans le plan A de l'extrémité de la tête 501. Dans la configuration représentée sur la figure 9, la tête 501 en étant appliquée par son extrémité A contre le matériau à étudier, permet de déterminer la couleur interne d'un matériau non opaque.

En faisant coulisser vers le bas la paroi extérieure 506, comme illustré sur la figure 10, on fait reculer le plan A d'extrémité du système émetteur 503 et du système détecteur 505, par rapport à l'extrémité de la paroi extérieure 506 qui sera appliquée contre le matériau. Dans une telle configuration, on effectuera une mesure colorimétrique de type classique portant essentiellement sur la couleur de surface du matériau.

La source de lumière S peut travailler dans la lumière visible, ou dans d'autres plages, par exemple en ultraviolet.

La tête optique de l'invention peut servir à déterminer la couleur interne de matériaux très divers, autres que la peau, par exemple des savons, savonnettes, ou des fruits, tels que des pommes, notamment pour déterminer si une pomme est talée ou non, des plastiques, des dents, tout matériau non opaque ou non parfaitement transparent.

La lumière recueillie par le système récepteur est analysée suivant une méthode classique, par exemple selon le système colorimétrique L*, a*, b*.

**L*** (généralement appelée clarté) désigne un résultat de mesure qui permet une quantification du blanc au noir (luminance).

**a*** est un chiffre de mesure qui, s'il est positif élevé, caractérise une composante très rouge.

**a*** négatif élevé caractérise une composante très verte.

**b*** est un chiffre de mesure qui, s'il est positif élevé, caractérise une composante très jaune.

**b*** négatif élevé caractérise une composante très bleue.

Les moyens d'analyse colorimétrique classiques ont été utilisés avec la tête de mesure selon l'invention, pour analyser la lumière recueillie par le système détecteur 5. Toutefois, les trois composantes données par ces moyens d'analyse colorimétrique ne correspondent plus exactement aux composantes L*, a*, b*, du système classique puisque la tête optique a été modifiée. On désignera par T, α et β les trois composantes obtenues avec une tête selon l'invention.

Des mesures ont été effectuées sur deux types de peaux humaines comprises entre très claire et claire, qui présentaient un aspect visuel différent.

Une première série de mesures sur ces deux peaux a été effectuée avec un colorimètre classique fabriqué par la société "Minolta" sous la référence CR 200. Les résultats obtenus sont les suivants :

| **L*** | **a*** | **b*** |
|---|---|---|
| 70 | 6,2 | 16,6 |
| 70,1 | 6 | 16,5 |

L'incertitude absolue sur les valeurs numériques fournies par l'appareil étant de l'ordre de ± 0,3, on voit que les valeurs fournies pour ces deux peaux, d'aspect visuel différent, par le colorimètre classique ne permettent pas de les distinguer.

Avec le colorimètre selon l'invention, on a obtenu les résultats suivants :

| **T** | α | β |
|---|---|---|
| 34,8 | 29 | 40,8 |
| 31,6 | 27,1 | 37,9 |

A la lecture de ces chiffres, il apparaît que, avec le colorimètre selon l'invention, on effectue une distinction entre les deux peaux. Ainsi, selon l'invention, on peut déterminer la couleur interne d'un matériau non opaque en appliquant contre ce matériau la tête de mesure colorimétrique qui présente une surface épousant la surface du matériau, et qui comporte un émetteur de lumière séparé d'un récepteur par une paroi opaque.

## Revendications

1. Tête de mesure colorimétrique, pour déterminer la couleur interne d'un matériau non opaque, en particulier la couleur interne de la peau humaine, comportant un système optique émetteur de lumière (503), en direction du matériau à étudier, séparé par une paroi opaque (504) d'un système optique (505) récepteur de la lumière qui a pénétré dans le matériau et qui est renvoyée par celui-ci, la surface externe (A) de la tête tournée vers le matériau à étudier (2) étant continue et adaptée à la surface du matériau de manière à épouser cette surface, caractérisée par le fait qu'elle comporte une paroi opaque extérieure (506) coulissante entre une position en saillie pour permettre une mesure classique de couleur de surface, et une position effacée où l'extrémité de la paroi opaque (506) est située sur la surface continue (A) de l'extrémité de la tête, de manière à s'appliquer contre le matériau et à permettre la mesure de la couleur interne de ce matériau non opaque.

2. Tête selon la revendication 1, caractérisée par le fait qu'elle est entourée d'une paroi opaque (506) protégeant la tête contre de la lumière extérieure parasite.

3. Tête selon la revendication 1 ou 2, caractérisée par le fait que l'émetteur (3a) est propre à émettre un faisceau de lumière parallèle.

4. Tête selon la revendication 1 ou 2, caractérisée par le fait que l'émetteur (3b, 103) émet de la lumière diffuse.

5. Tête selon l'une des revendications précédentes, caractérisée par le fait que le détecteur (405) assure une détection multidirectionnelle.

6. Tête selon la revendication 5, caractérisée par le fait que le récepteur comprend une sphère d'intégration (405).

7. Tête selon l'une des revendications 1 à 4, caractérisée par le fait que le détecteur (205, 305) assure une détection collimatée.

8. Tête selon la revendication 7, caractérisée par le fait qu'elle comporte un système collimaté (C) qui comprend, au niveau du détecteur (305), une lentille convergente (8) dont la face avant est dans le plan (A) de la tête (301) destiné à s'appliquer contre le matériau, un écran (9) étant disposé avec un orifice (10) en son centre, situé au foyer de la lentille (8), une cellule de détection (11) étant placée derrière l'orifice (10).

9. Procédé pour déterminer la couleur interne d'un matériau non opaque, caractérisée par le fait que l'on applique contre ce matériau une tête de mesure colorimétrique (1, 101, 201, 301, 401, 501) présentant une surface (A) qui épouse la surface du matériau, la tête de mesure comportant un émetteur de lumière (3, 103, 203, 303, 403, 503) séparé d'un récepteur (5, 105, 205, 305, 405, 505) par une paroi opaque (4, 104, 204, 304, 404, 504), et une paroi opaque extérieure (506) coulissante entre une position en saillie pour permettre une mesure classique de couleur de surface, et une position effacée où l'extrémité de la paroi opaque (506) est située sur la surface continue (A) de l'extrémité de la tête, de manière à s'appliquer contre le matériau et à permettre la mesure de la couleur interne de ce matériau non opaque.

10. Procédé selon la revendication 9, caractérisé par le fait que la surface externe (A) de la tête épouse la surface du matériau à étudier soit par application directe contre ladite surface du matériau, soit avec un mouillage optique.

## Claims

1. Colorimetric measurement head, for determining the internal colour of a non-opaque material, in particular the internal colour of the human skin, including an optical system (503) which emits light towards the material to be studied and is separated by an opaque wall (504) from an optical system (505) which receives light which has penetrated into the material and which is reflected thereby, the external surface (A) of the head, which is turned towards the material (2) to be studied, being continuous and adapted to the surface of the material so as to match this surface, characterized in that it includes an outer opaque wall (506) which slides between a projecting position for allowing conventional surface colour measurement, and a retracted position in which the end of the opaque wall (506) is located on the continuous surface (A) of the end of the head, so as to be applied against the material and to allow measurement of the internal colour of this non-opaque material.

2. Head according to Claim 1, characterized in that it is surrounded by an opaque wall (506) protecting the head against stray external light.

3. Head according to Claim 1 or 2, characterized in that the emitter (3a) is capable of emitting a parallel light beam.

4. Head according to Claim 1 or 2, characterized in that the emitter (3b, 103) emits diffuse light.

5. Head according to one of the preceding claims, characterized in that the detector (405) provides multidirectional detection.

6. Head according to Claim 5, characterized in that the receiver comprises an integration sphere (405).

7. Head according to one of Claims 1 to 4, characterized in that the detector (205, 305) provides collimated detection.

8. Head according to Claim 7, characterized in that it includes a collimated system (C) which comprises, at the level of the detector (305), a converging lens (A) whose front face is in the plane (A) of the head (301) which is intended to be applied against the material, a screen (9) being arranged with an aperture (10) at its centre, located at the focus of the lens (8), a detection cell (11) being placed behind the aperture (10).

9. Method for determining the internal colour of a non-opaque material, characterized in that a colorimetric measurement head (1, 101, 201, 301, 401, 501) having a surface (A) which matches the surface of the material is applied against this material, the measurement head including a light emitter (3, 103, 203, 303, 403, 503) separated from a receiver (5, 105, 205, 305, 405, 505) by an opaque wall (4, 104, 204, 304, 304, 404, 504) and an outer opaque wall (506) which slides between a projecting position for allowing conventional surface colour measurement, and a retracted position in which the end of the opaque wall (506) is located on the continuous surface (A) of the end of the head, so as to be applied against the material and to allow measurement of the internal colour of this non-opaque material.

10. Method according to Claim 9, characterized in that the outer surface (A) of the head matches the surface of the material to be studied either by direct application against the said surface of the material, or by an optical wetting.

## Patentansprüche

1. Kolorimetrischer Meßkopf zur Bestimmung der inneren Farbe eines nicht-lichtundurchlässigen Materials, insbesondere der inneren Farbe der menschlichen Haut, mit einem optischen Systems (503) zur Emission von Licht in Richtung des zu untersuchenden Materials, das durch eine lichtundurchlässige Wand (504) von einem optischen Empfangssystems (505) für das Licht getrennt ist, welches in das Material eingedrungen und von diesem zurückgeworfen wurde, wobei die zu dem zu untersuchenden Material (2) gerichtete Außenfläche (A) des Kopfes kontinuierlich und an die Oberfläche des Materials so angepaßt ist, daß sie sich dieser Oberfläche anschmiegt, dadurch gekennzeichnet, daß er eine lichtundurchlässige Außenwand (506) aufweist, die zwischen einer vorspringenden Position, welche eine herkömmliche Messung der Oberflächenfarbe erlaubt, und einer zurückgezogenen Position verschiebbar ist, in welcher sich das Ende der lichtundurchlässigen Wand (506) auf der kontinuierlichen Oberfläche (A) des Endes des Kopfes befindet, so daß er sich gegen das Material preßt und eine Messung der inneren Farbe des nicht-lichtundurchlässigen Materials erlaubt.

2. Kopf gemäß Anspruch 1, dadurch gekennzeichnet, daß er von einer lichtundurchlässigen Wand (506) umgeben ist, die den Kopf gegen Streulicht schützt.

3. Kopf gemäß Anspruch 1 oder 2, dadurch gekennzeichnet, daß der Emitter (3a) einen parallelen Lichtstrahl aussenden kann.

4. Kopf gemäß Anspruch 1 oder 2, dadurch gekennzeichnet, daß der Emitter (3b, 103) diffuses Licht aussendet.

5. Kopf gemäß einem der vorhergehenden Ansprüche, dadurch gekennzeichet, daß der Detektor (405) eine multidirektionelle Detektion ermöglicht.

6. Kopf gemäß Anspruch 5, dadurch gekennzeichnet, daß der Empfänger ein Kugelphotometer (405) umfaßt.

7. Kopf gemäß einem der Ansprüche 1 bis 4, dadurch gekennzeichnet, daß der Detektor (205, 305) eine kollimierte Detektion gewährleistet.

8. Kopf gemäß Anspruch 7, dadurch gekennzeichnet, daß er eine Kollimationssystem (C) aufweist, mit einer auf Höhe des Detektors (305) befindlichen Sammellinse (8), deren Stirnfläche sich in der Ebene (A) des Kopfes (301) befindet, welche gegen das Material gepreßt werden soll, einer mit einer zentralen Öffnung (10) versehenen Blende (9), die im Brennpunkt der Linse (8) angeordnet ist, und einer hinter der Öffnung (10) angeordneten Detektionszelle (11).

9. Verfahren zur Bestimmung der inneren Farbe eines nichtlichtundurchlässigen Materials, dadurch gekennzeichnet, daß man einen kolorimetrischen Meßkopf (1, 101, 201, 301, 401, 501) gegen das Material preßt, welcher eine sich an die Materialoberfläche angeschmiegende Fläche (A) aufweist, wobei der Meßkopf einen Lichtemitter (3, 103, 203, 303, 403, 503), der durch eine lichtundurchlässige Wand (4, 104, 204, 304, 404, 504) von einem Empfänger (5, 105, 205, 305, 405, 505) getrennt ist, und eine lichtundurchlässige Außenwand (506) aufweist, die zwischen einer vorspringenden Position, die eine herkömmliche Messung der Oberflächenfarbe ermöglicht, und einer zurückgezogenen Position verschiebbar ist, in welcher das Ende der lichtundurchlässigen Wand (506) sich auf der kontinuierlichen Fläche (A) des Endes des Kopfes befindet, so daß er gegen das Material gepreßt wird und die Messung der inneren Farbe des nicht-lichtundurchlässigen Materials ermöglicht.

10. Verfahren gemäß Anspruch 9, dadurch gekennzeichnet, daß die Außenfläche (A) des Kopfes sich der Oberfläche des zu untersuchenden Materials entweder durch direktes Andrücken gegen die Materialoberfläche oder mit optischer Benetzung anpaßt.
